# EUROPÄISCHE PATENTSCHRIFT

(11) **EP 0 780 382 B1**
(45) Veröffentlichungstag und Bekanntmachung des Hinweises auf die Patenterteilung: **11.06.2003**
(21) Anmeldenummer: 96119936.1
(22) Anmeldetag: 12.12.1996
(51) Int. Cl.: C07D 251/22, A61K 7/42

(54) **S-Triazinederivate als Lichtschutzmittel für Kosmetika**
S-Triazine derivatives as sunscreen agents for cosmetics
Dérivés de s-triazine comme agents de protection contre la lumière pour des compositions cosmétiques

(30) Priorität: 19.12.1995 CH 358095; 20.09.1996 CH 230596
(43) Veröffentlichungstag der Anmeldung: 25.06.1997
(73) Patentinhaber: F. HOFFMANN-LA ROCHE AG, 4070 Basel (CH)
(72) Erfinder: Huber, Ulrich, 8703 Erlenbach (CH)
(74) Vertreter: Keller, Günter, Dr.

(56) Entgegenhaltungen:
- DE-B- 1 241 452
- US-A- 3 896 125

## Beschreibung

Es ist bekannt, dass Sonnenlicht die Alterung der Haut beschleunigt, und sogar Hautkrebs hervorruft, und zwar werden diese unerwünschten Effekte vor allem durch die die Haut direkt bräunende UV A-Strahlung mit Wellenlängen im Bereiche von etwa 320 bis 400 nm verursacht.

Die amerikanische Patentschrift US-A-3896125 und die deutsche Auslegeschrift DE-A-1241452 beschreiben eine Vielzahl von o-Hydroxyphenyl-Verbindungen, die als Lichtschutzmittel eingesetzt werden können. Beide Veröffentlichungen umfassen generisch die Verbindungen der vorliegenden Erfinding. Das Problem der ausreichenden Löslichkeit solcher Verbindungen in kosmetischen Lösungsmitteln wird in keiner Veröffentlichung angesprochen.

Es wurde nun gefunden, dass die Verbindungen der Formel 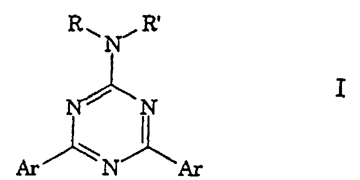 worin R und R' Butyl, Isobutyl, Pentyl, Isopentyl, Octyl oder 2-Ethylhexyl sind, und
Ar einer der Reste 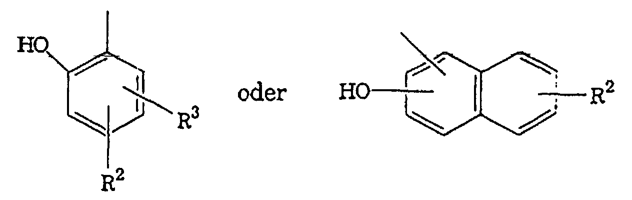 darstellt, worin R² Hydroxy, Methyl, oder einen gegebenenfalls durch eine oder mehrere Hydroxy- und/oder Alkoxygruppen substituierter Alkyl-, Alkenyl-, Alkinyl-etherrest oder Polyalkyletherrest darstellt, und R³ Wasserstoff ist oder einen Rest R² bedeutet, und worin die Hydroxygruppe in α-Stellung zu der Bindung zum Triazinkern steht,
vorzügliche UV A-Filter darstellen, indem sie bei ausgezeichneter Hautverträglichkeit und Stabilität (Licht, Hitze, Feuchtigkeit) eine starke Reduktion der Stresswirkung auf die Haut und damit eine Verzögerung der Hautalterung bewirken. Insbesondere weisen diese UV-A Filter aber auch eine ausgezeichnete Photostabilität auf.

Beispiele von
Alkylresten sind C₁ - C₂₀-Alkyle, wie Methyl, Ethyl, Propyl, Isopropyl, n-Butyl, sec.Butyl, Isobutyl, Pentyl, Isopentyl, n-Hexyl, n-Octyl, 2-Ethylhexyl, Iso-nonyl, n-Docecyl, Hexadecyl, Cocoyl, Eicosyl, 3,7,11-Trimethyldodecyl,
von Alkenylresten sind C₂ - C₁₂-Alkenyle, wie Allyl, Propenyl, Methylpropenyl, ω-Undecenyl, 2,4-Dodecadienyl, 3,7-Dimethyl-2,6-heptadienyl,
von Alkinylresten sind C₂ - C₁₂-Alkinyle, wie Ethinyl, Propargyl, 2-methyl-4-pentinyl, ω-Undecinyl, 3-Dodecinyl,
von Alkyletherresten sind C₂ - C₂₀-Alkyletherreste, wie 2-Ethoxy-ethyl, 2-Methoxy-1-methyl-ethoxy, 3-Methyloxy-propyl, 3-(2-Ethyl-hexyl)-oxy-propyl, 2-Ethoxybutyl, Methoxymethyl, 2-Ethoxy-octadecanyl,
von Alkenylätherresten und Alkinylätherresten sind Allyl- und Propargyl,
von Polyoxalkyletherresten sind C₄ - C₂₀-Reste, wie 2-[2-(2-Methoxy)-ethoxy]-ethyl, 2-[2-(2-Hydroxy)ethoxy]-ethyl, 2-(2-Ethoxy-1-methyl-ethoxy)-1-methyl-ethyl, 2-(2-{2-[2-(2-Hydroxy-propoxy)-propoxy]-propoxy}-propoxy)-propyl, 2-(2-{2-[2-(2-(2-Ethoxy-propoxy)-propoxy)-propoxy]-propoxy}-propoxy)-propyl, 2-(2-{2-[2-(2-(2-Hydroxy-ethoxy)-ethoxy)-ethoxy]-ethoxy}-ethoxy)-ethyl,

Beispiele von Resten Ar sind:

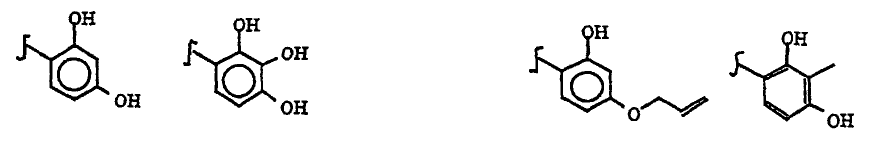

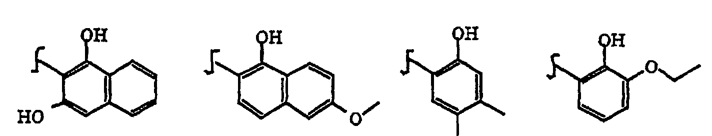

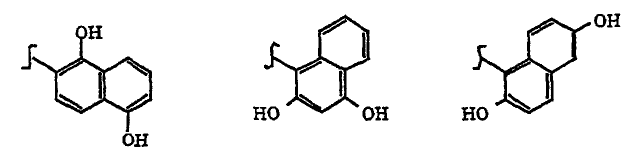

Es wurde weiter gefunden, dass die Verbindungen der Formel I die Schutzwirkung von UV B-Filtern, d.h. von Substanzen, welche die erythemerzeugende UV-B-Strahlung in Bereiche von etwa 290 bis 320 nm absorbieren, überraschenderweise erhöhen, obwohl das Absorptionsmaximum der Verbindung I nicht in diesem Bereich, sondern im Bereich von etwa 320 bis 360 nm liegt.

Gegenstand der Erfindung sind demgemäss die neuen Verbindungen I, deren Herstellung, Lichtschutzmittel, nämlich Lichtschutzpräparate für kosmetische Zwecke, mit einem Gehalt an Verbindungen der obigen Formel I, vorzugsweise in Kombination mit einem UV B-Filter, und die Verwendung der Verbindungen I als Lichtschutzmittel, insbesondere für kosmetische Zwecke.

Im letzteren Fall werden Lichtschutzpräparate erhalten, die die UV-Strahlung im gesamten Bereich von 280 bis 380 nm absorbieren - einen sogenannten "A+B-Total-block"darstellen - und die Haut vor zu früher Alterung und in vielen Fällen vor Lichtdermatosen schützen.

Die Herstellung dieser neuen Lichtschutzmittel (insbesondere von Hautschutzpräparaten für die Alltagskosmetik) geschieht dadurch, dass man die Verbindung der Formel I, vorzugsweise in Kombination mit einem UV B-Filter, in eine für Lichtschutzmittel übliche kosmetische Grundlage einarbeitet.

Als UV B-Filter im Sinne der vorliegenden Erfindung, d.h. als Substanzen mit Absorptionsmaxima zwischen etwa 290 und 320 nm, können übliche UV B-Filter, beispielsweise die nachstehenden, zu den verschiedensten Stoffklassen gehörenden, organischen Verbindungen genannt werden:
1) Derivate der p-Aminobenzoesäure, wie Ethyl-p-aminobenzoat und andere Ester, wie Propyl-, Butyl-, Isobutyl-p-aminobenzoat. Ethyl-pdimethylaminobenzoat, Glyceryl-p-aminobenzoat, Amyl-p-dimethylaminobenzoat, Octyl-p-N,N- dimethyl-aminobenzoat,
2a) Derivate der Zimtsäure, wie 2-Ethoxyethyl-p-methoxyzimtsäureester, 2-Ethylhexyl(oder Pentyl)-p-methoxyzimtsäure-ester, p-Methoxyzimtsäureestergemische, Zimtsäureestergemische,
2b) Derivate der Malonsäure, z.B. Phenylmethylen-malonsäureester und an Siloxane gebundene 2a) und 2b) z.B. die in EP 789 080 als "Polysiloxan A" bezeichneten Verbindungen,
3) Heterozyklische Stickstoffverbindungen, wie Derivate des 2-Phenylbenzimidazols, z.B. 2-Phenylbenzimidazol-5-sulfonsäure,
4) Derivate der Salicylsäure, z.B. Salicylsäuremethylester, Salicylsäure-homomenthylester, Salicylsäure-phenylester, Salicylsäureethylhexylester,
5) Derivate des Benzophenons, wie 4-Phenylbenzophenon, 4-Phenylbenzophenon-2-carbonsäure-isooctylester, 2-hydroxy-4-methoxybenzophenon-5-sulfonsäure, 2,2'-Dihydroxy-4-methoxybenzophenone, Bis(2,4-dihydroxyphenyl)methanone, 2-Hydroxy-4-octoxybenzophenone,
6) Derivate der Gallussäure, wie Digalloyl-trioleat,
7) Arylidencycloalkanone, wie Benzylidencampher, p-tert.-Butylbenzylidencampher (bevorzugt) oder Methylbenzylidencampher,
8) Derivate der Anthranilsäure, wie Anthranilsäurementhylester,
9) Hydroxyphenylbenztriazol,
10) Acrylate, z.B. 2-Ethylhexyl-2-cyano-3,3-diphenylacrylat (Octocrylene), Ethyl-2-cyano-3,3-diphenylacrylat,
11) Mikrokristalline Pulver, z.B. mikrokristallines TiO₂, ZnO, Fe₂O₃.

Die unter 2) aufgeführten Verbindungen, ganz besonders der 2-Ethylhexyl-p-methoxyzimtsäureester, sind bevorzugt.

Als für Lichtschutzmittel übliche kosmetische Grundlage im Sinne der vorliegenden Erfindung kann jede übliche Zubereitung dienen, die den kosmetischen Anforderungen entspricht, z.B. Crèmes, Lotions, Emulsionen, Salben, Gele, Lösungen, Sprays, Sticks, Milch; siehe auch Kosmetik, Entwicklung, Herstellung und Anwendung Kosmetischer Mittel, ed. Wilfried Umbach, Georg Thieme Vertrag Stuttgart - New York 1988, Sunscreens, Development, Evaluation and Regulatory Aspects, ed. N.Y. Lowe, N.A. Shaath, Marcel Dekker, Inc. New York Basel, 1990.

Die Lichtschutzwirkung ist auch von der verwendeten Grundlage abhängig. Die Intensität der Lichtschutzwirkung hängt weiter bei gleicher Grundlage von der Wirkstoffkonzentration ab. Geeignete Konzentrationen sind z.B. 1-6%, vorzugsweise 2-5% der Verbindung der Formel I im kosmetischen Präparat. Das Verhältnis von Verbindung I zum UV B-Filter ist nicht kritisch. Aus ökonomischen Gründen beträgt es aber beispielsweise 1-2 Teile des UV B-Filters auf 1 Teil der Verbindung I.

Auf Grund ihrer Lipophilität lassen sich die Verbindungen I gut in ölund fetthaltige kosmetische Zubereitungen einarbeiten.

Bezüglich der Lipophilität sind die neuen Verbindungen den bekannten, industriellen Lichtschutzmitteln unter Verwendung von substituierten Triazinen, siehe USP 3 896 125 bzw. DAS 1 241 452 überlegen, indem alle I das im vorliegenden Fall nötige Kriterium, nämlich eine Löslichkeit von ≥ 1% in kosmetischen Lösungsmitteln, wie z.B. Cétiol LC (Cocoyl-caprylat/-caprat), Lexorez 100 (Glycerin diethylenglykoladipat Kreuzpolymer) oder Crodemol DA (Diisopropyladipat) erfüllen. Im Falle der vorbekannten Strukturen werden andererseits Löslichkeiten von nur 0,5% oder weniger erreicht.

Eine diesbezüglich besonders bevorzugte Verbindung ist die Verbindung des Beispiels 1.

Aber auch der strukturelle Aufbau (und damit die Zugänglichkeit) sind im vorliegenden Fall einfacher.

Die Verbindungen I sind neu. Sie bilden ebenfalls einen Gegenstand der vorliegenden Erfindung.

Der Zugang zu I kann wie folgt veranschaulicht werden:

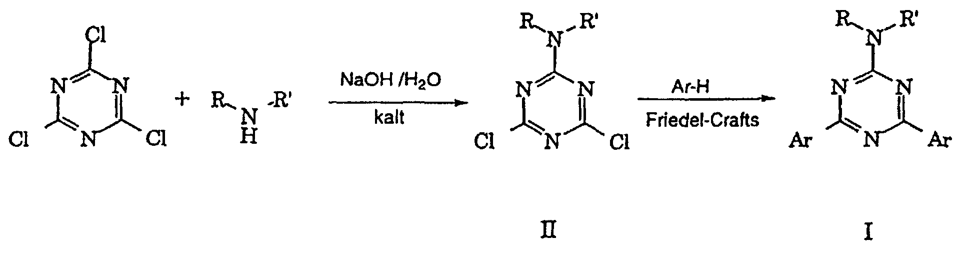

Das Verfahren von II → I ist also dadurch gekennzeichnet, dass man eine Verbindung der Formel 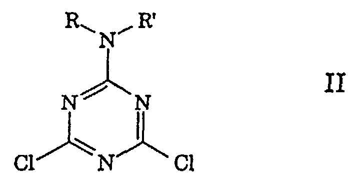 mit dem dem obigen Radikal Ar entsprechenden Grundkörper, z.B. unter den Bedingungen der Friedel-Crafts-reaktion umsetzt.

Freie Hydroxygruppen in R² und/oder R³ können gegebenenfalls noch verethert werden.

Die Umsetzung der Reaktionspartner kann unter den für die Friedel-Crafts-Reaktion üblichen Bedingungen, also in an sich bekannter Weise durchgeführt werden. Man arbeitet also mit oder ohne Lösungsmittel. Geeignete Lösungsmittel sind aprotische organische Lösungsmittel, z.B. Toluol, Nitrotoluol, Chlor-, Dichlor- und Trichlorbenzol, Kohlenwasserstoffe (z.B. Isooctan), chlorierte Kohlenwasserstoffe, Nitroalkane, Sulfolan (Tetramethylensulfon), Schwefelkohlenstoff, Schwefeldioxyd, etc. sowie Gemische davon. Es werden zweckmässigerweise die bekannten Katalysatoren benutzt, also Lewis-Säuren, wie Aluminiumchlorid, Zinnchlorid, Zinkchlorid, Titantetrachlorid, Bortrifluorid, etc. Der Temperaturbereich ist zweckmässigerweise derjenige von ca. 10°C bis ca. 220°C, vorzugsweise liegt er zwischen ca. 80 und 150°C.

Ein möglicher Zugang zu II ist oben illustriert, wobei die Methodik im Detail von J.T. Thurston et al., J. Amer. Chem. Soc. 73, 2981 (1951) beschrieben wird, und aus dem obenstehenden Schema und den untenstehenden Beispielen ersichtlich ist.

Die Veretherung freier Hydroxygruppen kann in an sich bekannter Weise erfolgen, z.B. indem man ein Phenol der allgemeinen Struktur I mit einem Ether bildenden Reagens, z.B. einem Alkylhalogenid (bzw. Alkenyl-, Alkinyl-halogenid), Alkyltosylat oder einem Epoxyd umsetzt. Die Reaktion kann ohne Lösungsmittel (bevorzugt) oder mit Lösungsmitteln (z.B. Diethylether, Ethanol, Aceton, DMSO, DMF, Toluol, etc), neutral, allenfalls unter Verwendung von Säuren (z.B. H₂SO₄, etc.), oder aber auch, vorzugsweise, mittels Basen (Pyridin, Trimethylamin, NaOH, NaOEt, tert.-Butyl-ONa, NaH, LiH, K, Na, K₂CO₃, Na₂CO₃, NaHCO₃, Butyllithium, etc.) umgesetzt werden.

Zusätzlich können Phasen-transferreagentien, z.B. Tetrabutylammoniumbromid, Kronenether, etc. eingesetzt werden.

Als Reagenzien kommen demnach z.B. in Frage: Methyliodid, Methylchlorid, Octylbromid, Allylchlorid, Propargylbromid, Ethylenoxyd, Propylenoxyd, Octan-1,2-epoxyd, Methyl-glycidylether, Octyl-glycidylether, etc.

Ein zweckmässiger Temperaturbereich ist derjenige von ca. 0°C bis ca. 150°C.

### Beispiel 1

### 4,4'-{6-[Bis-(2-ethyl-hexyl)-amino]-s-triazin-2,4-diyl}-diresorcin.

a) Herstellung von 4,6-Dichloro-[1,3,5]-triazin-2-yl-bis-(2-ethyl-hexyl)-amin: 18,4 g (0.1 Mol) Cyanurchlorid werden in 75 ml Aceton suspendiert und zu 90 ml gekühltem Wasser zugetropft. Darauf werden 24.1 g (0.1 Mol) Bis-(2-ethyl-hexyl)-amin und anschliessend 4 g (0.1 Mol) NaOH, gelöst in 14 ml Wasser, zugetropft. Die Suspension wird zwei Stunden bei 20°C gerührt, dann auf 200 ml Wasser gegossen, und mit Essigester extrahiert. Die getrockneten und eingeengten, vereinigten, organischen Phasen ergeben 35 g eines klaren Oels des gewünschten Dichlorotriazins, dessen Struktur mittels MS (388 = M+) nachgewiesen wird.
b) Eine Suspension von 16,5 g (0,124 Mol) AlCl₃ und 23.2 g (0.06 Mol) des oben erhaltenen Oels in 150 ml Toluol, wird auf 70°C erwärmt, und mit 14.5 g (0.132 Mol) Resorcin in 19 g Sulfolan versetzt. Dieses Gemisch wird während 24 Stunden bei 110°C gerührt und darauf zwischen Wasser und Essigester verteilt. Die Essigesterphase wird mit 2n NaOH-Lösung extrahiert, und der NaOH Extrakt mit verdünnter HCl angesäuert und filtriert. Das erhaltene Produkt wurd mit NaCl-Lösung neutral gewaschen und aus Hexan/Diethylether umkristallisiert.

Ausbeute 21.9 g eines feinen Pulvers des gewünschten Produktes. Schmelzpunkt 141-143°C, UV 339 nm (E=648).

### Beispiel 2

### 4,4'-[6-(Di-n-octyl-amino)-s-triazin-2,4-diyl]-diresorcin.

Es wird verfahren wie in Beispiel 1, jedoch wird ein Aequivalent Di-noctylamin statt Bis-(2-ethyl-hexyl)-amin eingesetzt.

Die Ausbeute der 1. Stufe beträgt 76% und der 2. Stufe 51% der Theorie. Smp.: 165-67°C. UV 338 nm (E=724).

### Beispiel 3

### 4,4'-[6-(Di-n-pentyl-amino)-s-triazin-2,4-diyl]-diresorcin.

Es wird verfahren wie in Beispiel 1, jedoch wird ein Aequivalent Di-npentylamin statt Bis-(2-ethyl-hexyl)-amin eingesetzt.

Die Ausbeute der 1. Stufe beträgt 81% und der 2. Stufe 46% der Theorie. Smp.: 190-92°C. UV 339 nm (E=681).

### Beispiel 4

### 4,4'-[6-(Di-isobutyl-amino)-s-triazin-2,4-diyl]-diresorcin.

Es wird verfahren wie in Beispiel 1, jedoch wird ein Aequivalent Diisobutylamin statt Bis(2-ethyl-hexyl)-amin eingesetzt. Die Ausbeute in der 1. Stufe beträgt 89% und in der 2. Stufe 63% der Theorie. Smp.: 210-20°C. UV 338 nm (E=736).

### Beispiel 5

### 4,4'-[6-(Allyl-2-ethyl-hexyl-amino)-s-triazin-2,4-diyl]-diresorcin.

Es wird verfahren wie in Beispiel 1, jedoch wird ein Aequivalent Allyl-2-ethyl-hexylamin statt Bis(2-ethyl-hexyl)-amin eingesetzt. Die Ausbeute der ersten Stufe beträgt 94% und der 2. Stufe 20% der Theorie. Smp.: 90-94°C. UV 340 nm (E= 896).

Das Allyl-2-ethyl-hexylamin wurde wie folgt hergestellt: 17.8 ml 2-Ethyl-hexyl-bromid werden bei 0°C zu 60 ml Allylamin zugetropft. Nun wird langsam auf Rückfluss erhitzt, 18 Stunden rückflussiert, und anschliessend das überschüssige Allylamin abdestilliert. Der Rückstand wird in Ether aufgenommen und mit wässriger NaOH und wässriger NaCl gewaschen, und wieder eingeengt. Man erhält 11.9 g (63%) eines gelblichen Oels von Allyl-2-ethyl-hexylamin, dessen Identität mit NMR, IR und MS bestätigt wurde.

### Beispiel 6

### 4,4'-{6-[Bis(2-ethyl-hexyl)-amino]-2,4-bis[4-(3-butoxy-2-hydroxy-propoxy)-2-hydroxy-phenyl]-[1,3,5]-triazin

2,15 g 4,4'-{6-[Bis-(2-ethyl-hexyl)-amino]-s-triazin-2,4-diyl}-diresorcin (hergestellt gemäss Beispiel 1) und 22 mg Tetrabutylammoniumchlorid werden in 40 ml Butylglycidether während 24 Stunden auf 130°C erhitzt. Danach wird die erkaltete, gelbe Lösung mit Wasser gewaschen, und am Wasserstrahlvacuum und anschliessend am Hochvacuum bei 0,05 Torr eingeengt und mit Hexan/Essigester über Kieselgel chromatographiert. Es werden 2.35 g eines gelblichen Oels erhalten, dessen Identität mittels NMR, IR und MS (796 = M+) bestätigt wird. UV 319 nm (E=346).

### Beispiel 7

### 4,4'-{6-[Bis(2-ethyl-hexyl)-amino]-2.4-bis(4-methyl-2-hydroxy-phenyl)-[1,3,5]triazin.

10 g des in Beispiel 1 beschriebenen Dichloro-triazins werden zusammen mit 6,1 g m-Kresol und 6,9 g AlCl₃ in 100 ml Xylol während 4 Tagen auf 120°C erhitzt. Nun wird das Reaktionsgemisch zwischen Wasser und Essigester verteilt, die organische Phase eingeengt und mit Hexan/Toluol durch Kieselgel chromatographiert. Ausbeute 1,9 g des obigen, kristallinen Produktes, Smp. 75-76°C, MS (532 = M+), UV 340 nm (E = 373).

### Beispiel 8

### 4,4'-{6-Bis-(2-ethyl-hexyl)-amino)-s-triazin-2,4-diyl}-di-naphthoresorcin.

Es wird verfahren wie in Beispiel 1, jedoch wird in b) 1,3-Naphthoresorcin statt Resorcin eingesetzt und das Reaktionsgemisch 18 Stunden auf 130°C erwärmt. Nach der Aufarbeitung wird das Rohprodukt in Essigester/Hexan = 1:1 über Kieselgel chromatographiert.

Man erhält das gewünschte Produkt: Smp. 100-105°C. UV 382 nm (E=384).

### Beispiel 9

### 4,4'-{6-(Dibutyl-amino)-2,4-bis(2,4-dihydroxy-5-hexyl-phenyl)-(1,3,5)-triazin.

Es wird verfahren wie in Beispiel 1, jedoch wird in a) Di-n-butylamin anstelle von Bis-(2-ethyl-hexyl)-amin eingesetzt und in b) wird 4-Hexylresorcin anstelle von Resorcin eingesetzt. Es wird 2 Tage auf 110°C erwärmt. Nach der Aufarbeitung wird das Rohprodukt aus Toluol/ Ethanol kristallisiert.

Man erhält das gewünschte Produkt: Smp. 203-206°C. UV 350 nm (E=350).

### Beispiel 10

### 4,4'-{6-(Bis-(2-ethyl-hexyl)-amino)-2,4-bis(2,4-dihydroxy-3-methylphenyl)-(1,3,5)-triazin.

Es wird verfahren wie in Beispiel 1, jedoch wird in b) 1,6-Dihydroxytoluol anstelle von Resorcin eingesetzt und die Reaktion 2 Stunden auf 130°C erwärmt. Nach der Aufarbeitung wird das Rohprodukt zweimal in Acetonitril umkristallisiert.

Man erhält das gewünschte Produkt: Smp. 90-93°C. UV 340 nm (E=653).

### Beispiel 11

### 4,4'-{6-(N-Isopentyl-N-3,5,5-trimethyl-hexyl-amino)-s-triazin-2,4-diyl}diresorcin.

Es wird verfahren wie in Beispiel 1, jedoch wird in a) N-Isopentyl-N-3,5,5-trimethyl-hexyl-amin anstelle von Bis-(2-ethyl-hexyl)-amin eingesetzt.

Man erhält das oben genannte Produkt in 52% Ausbeute: Smp. 187-189°C. UV 337,5 nm (E=665).

[N-Isopentyl-N-3,5,5-trimethyl-hexyl-amin ist aus "Isononylamin" (Höchst) und Isovaleriansäure-chlorid in wässeriger KOH bei 0-20°C, und anschliessender Reduktion mit LiAlH₄ in Ether unter Rückflusstemperatur zugänglich.]

### Beispiel 11

| Sonnenschutz Emulsion (o/w) | |
|---|---|
| | Gewichtsteile |
| A | |
| Parsol MCX (Octyl methoxycinnamat) | 5.0 |
| Triazin des Beispiels 1 | 3.0 |
| Cetyl-alcohol | 1.00 |
| Diethylenglycol-monostearat | 0.25 |
| Cetiol LC (Cocoyl-capryl/caprat) | 7.00 |
| MPOB/PPOB 70/30 (Methyl + Propylparaben- | 0.25 |
| ester der 4-Hydroxybenzoesäure) | |
| EDTA-Na₂ und | 0.1 |
| Amphisol K (Kalium cetyl phosphat) | 1.00 |
| | |
| werden in Reaktor zusammengegeben, geschmolzen und darauf bei 90°C versetzt mit einem Gemisch von | |

| B | |
|---|---|
| Pemulen TR-1 1% (C10-C30 Alkyl-acrylat-kreuzpolymer) | 20.0 |
| Wasser | 56.4 |
| Propylenglykol | 5.00 ; |
| schliesslich werden | |
| KOH 10% | 0.80 |
| und | |
| | |

| C | |
|---|---|
| eine Riechstoffkomposition | 0.20 |
| bei 40°C zugemischt. | |

### Beispiel 12

| Silicon-Wasser-Emulsion (Lotion) | |
|---|---|
| | Gewichtsteile |
| A | |
| Gilugel SIL5 (Cyclomethicone und Aluminium/Magnesium-hydroxid-stearat) | 5.0 |
| wird im Reaktor geschmolzen und versetzt mit einen Gemisch von | |

| B | |
|---|---|
| Silicone 3225 C (Siliconoel) | 10.0 |
| Silicone 245 (Cyclomethicone) | 5.0 |
| Witconol PPM (PPG-3 Myristyl ether) | 2.0 |
| und Parsol MCX | 5.0 |
| sowie anschliessend mit einem Gemisch von | |

| C | |
|---|---|
| Triazin des Beispiels 1 | 2.0 |
| Finsolv TN (C12-15-Alkyl benzoat) | 5.0 |
| Phenonip (Phenoxyethanol & Methylparaben & | 0.6 und |
| Ethylparaben & Propylparaben & Butylparaben) | |
| BHT (Butylhydroxytoluol) | 0.05 |
| EDTA-Na₂ | 0.1 |
| sowie mit | |
| Wasser | 58.85 |
| und darin gelöstem | |
| NaCl | 0.5. |
| Dieses Gemisch wird in Wasserbad auf 85°C erwärmt und versetzt mit | |

| E | |
|---|---|
| TiO2 | 1.9 |
| und einem Gemisch von | |
| Hyasol (Natrium Hyaluronat) | 2.0 |
| und Vitamin E Acetat (Tocopheryl-acetat) | 2.0 |

### Beipiel 13

| Sonnenschutz-Lotion (w/o) | |
|---|---|
| | Gewichtsteile |
| A | |
| Parsol MCX | 5.0 |
| Triazin des Beispiels 1 | 3.0 |
| Arlacel 481 (Glycerin/Sorbitan-Fettsäureester) | 9.0 |
| Elfacos C26 (Hydroxyoctacosanyl hydroxystearat) | 5.0 |
| Petroleum special (Petrolatum) | 2.0 |
| Vaselineöl | 10.0 |
| Cetiol LC | 10.0 |
| EDTA-Na₂ | 0.1 |
| und | |
| Phenonip | 0.6 |
| werden in einem Reaktor gemischt und geschmolzen; | |

| B | |
|---|---|
| je ein Gemisch von | |
| Wasser | 21.5 |
| Sorbit 70% | 5.0 |
| in Glycerin | 3.0 |
| und ein Gemisch von | |

| C | |
|---|---|
| Parsol HS (Phenylbenzimidazol-sulfonsäure) | 2.00 |
| KOH 10% | 4.29 |
| in Wasser | 19.31 |
| wird bei 90°C nacheinander zum ursprünglichen Gemisch zugegeben. | |
| Schliesslich wird bei 40°C | |
| Sunmild 718 (eine Riechstoffkomposition) | 0.2 |
| zugemischt. | |

## Patentansprüche

1. Verbindungen der Formel 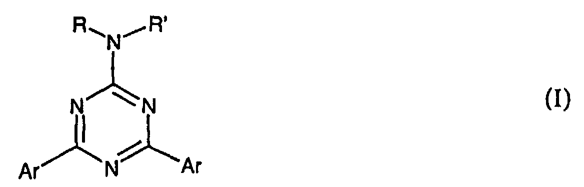 worin R und R' Butyl, Isobutyl, Pentyl, Isopentyl, Octyl oder 2-Ethylhexyl sind, und Ar einen der Reste 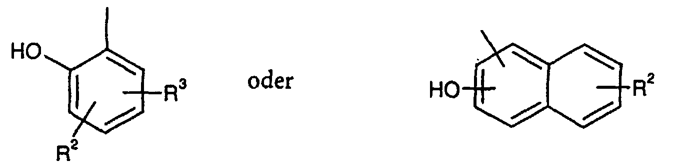 darstellt, worin R² Hydroxy, Methyl oder einen gegebenenfalls durch eine oder mehrere Hydroxy- und/oder Alkoxygruppen substituierten Alkyl-, Alkenyl, Alkinyl-etherrest oder Polyalkyletherrest darstellt, und R³ Wasserstoff ist oder einen Rest R² bedeutet, und worin die Hydroxygruppe in α-Stellung zu der Bindung zum Triazinkern steht.

2. 4,4'{6-[Bis(2-Ethyl-hexyl)-amino]-s-triazin-2,4-diyl}-diresorcin.

3. 4,4'[6-(Di-isobutyl-amino)-s-triazin-2,4-diyl]-diresorcin.

4. 4,4'-[6-(Di-n-octyl-amino)-s-triazin-2,4-diyl]-diresorcin.

5. 4,4'-[6-(Di-n-pentyl-amino)-s-triazin-2,4-diyl]-diresorcin.

6. 4,4'-{6-[Bis(2-ethyl-hexyl)-amino]-2,4-bis[4-(3-butoxy-2-hydroxy-propoxy)-2-hydroxy-phenyl]-[1,3,5]-triazin.

7. 4,4'-{6-[Bis(2-ethyl-hexyl)-amino]-2,4-bis(4-methyl-2-hydroxy-phenyl)-[1,3,5]-triazin.

8. 4,4'-{6-Bis-(2-ethyl-hexyl)-amino)-s-triazin-2,4-diyl}-di-naphthoresorcin.

9. 4,4'-{6-(Bis-(2-ethyl-hexyl)-amino)-2,4-bis(2,4-dihydroxy-3-methyl-phenyl)-(1,3,5)-triazin.

10. 4,4'-{6-(N-Isopentyl-N-3,5,5-trimethyl-hexyl-amino)-s-triazin-2,4-diyl}-diresorcin.

11. Lichtschutzmittel, **gekennzeichnet durch** einen Gehalt an einer Verbindung der Ansprüche 1 bis 10.

12. Verfahren zur Herstellung der Verbindungen der Formel 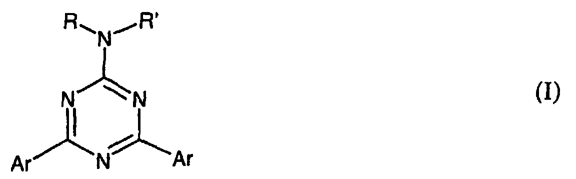 worin R und R' und Ar die in Anspruch 1 angegebene Bedeutung haben,
dadurch gegekennzeichnet, dass man eine Verbindung der Formel 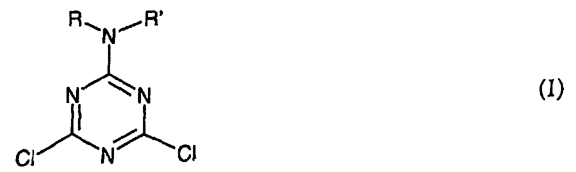 mit dem dem obigen Radikal Ar entsprechenden Grundkörper under den Bedingungen der Friedel-Crafts-Reaktion umsetzt, und freie Hydroxy-gruppen in R² und/oder R³ (oder "im Radikal Ar") gegebenenfalls veräthert.

13. Verwendung der Verbindungen der Ansprüche 1 bis 10 als Lichtschutzmittel.

## Claims

1. Compounds of the formula 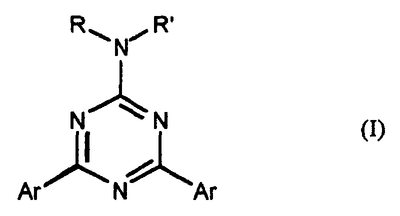 wherein R and R' are butyl, isobutyl, pentyl, isopentyl, octyl or 2-ethylhexyl and Ar represents one of the residues 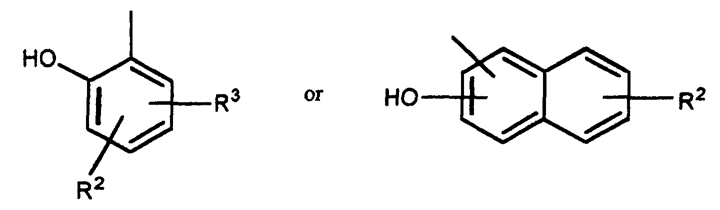 in which R² is hydroxy, methyl or an alkyl, alkenyl, alkynyl ether residue or polyalkyl ether residue optionally substituted by one or more hydroxy and/or alkoxy groups and R³ is hydrogen or signifies a residue R² and in which the hydroxy group is present in the α-position to the bonding to the triazine nucleus.

2. 4,4'-{6-[Bis(2-ethyl-hexyl)-amino]-s-triazine-2,4-diyl}-diresorcinol.

3. 4,4'-[6-(Di-isobutyl-amino)-s-triazine-2,4-diyl]-diresorcinol.

4. 4,4'-[6-(Di-n-octyl-amino)-s-triazine-2,4-diyl]-diresorcinol.

5. 4,4'-[6-(Di-n-pentyl-amino)-s-triazine-2,4-diyl]-diresorcinol.

6. 4,4'-{6-[Bis(2-ethyl-hexyl)-amino]-2,4-bis[4-(3-butoxy-2-hydroxy-propoxy)-2-hydroxy-phenyl]-[1,3,5] -triazine.

7. 4,4'-{6-[Bis(2-ethyl-hexyl)-amino]-2,4-bis(4-methyl-2-hydroxy-phenyl)-[1,3,5]-triazine.

8. 4,4'-{6-Bis-(2-ethyl-hexyl)-amino)-s-triazine-2,4-diyl}-di-naphthoresorcinol.

9. 4,4'-{6-(Bis-(2-ethyl-hexyl)-amino)-2,4-bis(2,4-dihydroxy-3-methyl-phenyl)-(1,3,5)-triazine.

10. 4,4'-{6-(N-Isopentyl-N-3,5,5-trimethyl-hexyl-amino)-s-triazine-2,4-diyl}-diresorcinol.

11. A light screening agent, **characterized by** a content of a compound of claims 1-10.

12. A process for the manufacture of the compounds of the formula 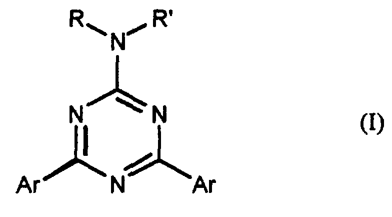 wherein R and R' and Ar have the significance given in claim 1,
**characterized by** reacting a compound of the formula 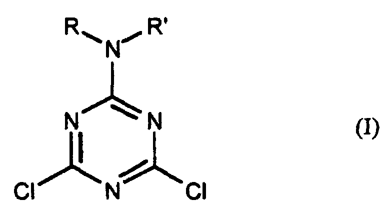 with the substance corresponding to the above radical Ar under the conditions of the Friedel-Crafts reaction and, if desired, etherifying free hydroxy groups in R² and/or R³ (or "in the radical Ar").

13. The use of the compounds of claims 1 to 10 as light screening agents.

## Revendications

1. Composés de formule 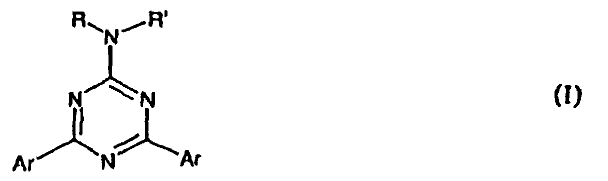 dans laquelle R et R' représentent le groupe butyle, isobutyle, pentyle, isopentyle, octyle ou 2-éthylhexyle, et
Ar représente l'un des radicaux 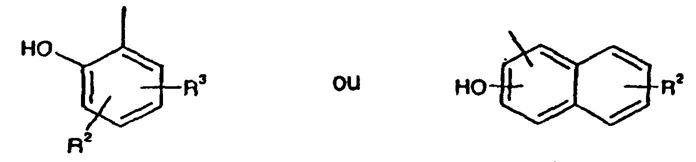 où R² représente le groupe hydroxy ou méthyle ou un radical alkyleéther, alcényleéther, alcynyleéther éventuellement substitué par un ou plusieurs groupes hydroxy et/ou alcoxy, ou un radical polyalkyléther, et R³ représente un atome d'hydrogène ou un radical R², et où le groupe hydroxy est en position a par rapport à la liaison du noyau triazine.

2. 4,4'-[6-[bis(2-éthylhexyl)-amino]-s-triazine-2,4-diyl]-dirésorcinol.

3. 4,4'-[6-(di-isobutyl-amino)-s-triazine-2,4-diyl]-dirésorcinol.

4. 4,4'-[6-(di-n-octyl-amino)-s-trlazine-2,4-diyl]-dirésorcinol.

5. 4,4'-[6-(di-n-pentyl-amino)-s-triazine-2,4-diyl]-dirésorcinol.

6. 4,4'-[6-[bis(2-éthylhexyl)-amino]-2,4-bis[4-(3-butoxy-2-hydroxy-propoxy)-2-hydroxyphényl]-[1,3,5]-triazine.

7. 4,4'-[6-[bis(2-éthylhexyl)-amino]-2,4-bis(4-méthyl-2-hydroxyphényl)-[1,3,5]-triazine.

8. 4,4'-[6-[bis(2-éthylhexyl)-amino]-s-triazine-2,4-diyl]-dinaphtorésorcinol.

9. 4,4'-[6-[bis(2-éthylhexyl)-amino]-2,4-bis(2,4-dihydroxy-3-méthylphényl)-(1,3,5)-triazine.

10. 4,4'-[6-(N-isopentyl-N-3,5,5-triméthyl-hexyl-amino)-s-triazine-2,4-diyl]-dirésorcinol.

11. Photoprotecteur **caractérisé par** une teneur en un composé des revendications 1 à 10.

12. Procédé pour la préparation des composés de formule 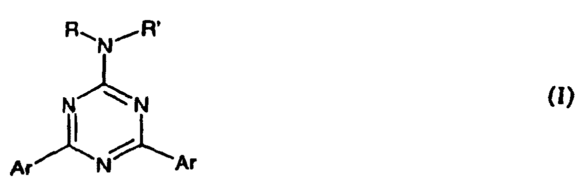 dans laquelle R et R' et Ar ont les significations données dans la revendication 1, **caractérisé en ce qu'**on fait réagir un composé de formule 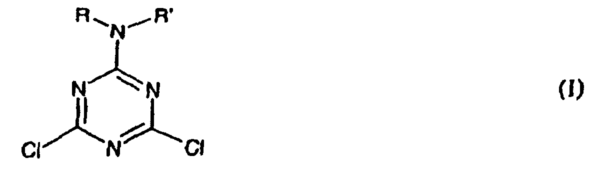 avec le corps de base correspondant au radical Ar ci-dessus, dans les conditions de la réaction de Friedel-Crafts, et éventuellement on éthérifie des groupes hydroxy libres dans R² et/ou R³ (ou "dans le radical Ar").

13. Utilisation des composés des revendications 1 à 10 en tant que photoprotecteur.
